# EUROPEAN PATENT APPLICATION

(11) **EP 1 225 181 A1**
(43) Date of publication of application: **24.07.2002**
(21) Application number: 01100762.2
(22) Date of filing: 12.01.2001
(51) Int. Cl.: C07K 1/22

(54) **Protein expression and structure resolution using specific fusion vectors**

(71) Applicant: Max-Planck-Gesellschaft zur Förderung der Wissenschaften e.V., 80539 München (DE)
(72) Inventor: Manstein, Dietmar J,Dr, 69121 Heidelberg (DE); Kull, Jon F, Dr., 69120 Heidelberg (DE); Knetsch, Menno L.W, Dr., 69126 Heidelberg (DE); Niemann, Hartmut H., 69118 Heidelberg (DE)
(74) Representative: Graf von Stosch, Andreas, Dr. rer. nat.

(57) **Abstract**

The present invention relates to a recombinant protein composed of a catalytic domain of a motor protein such as kinesin or myosin, a linker sequence and a protein of interest, a DNA sequence encoding such a recombinant protein, a vector expressing such a recombinant protein, a host cell transformed with a vector, a method for producing a recombinant protein, and methods for purification, crystallization and structure elucidation of the recombinant protein.

### Annex to the application documents - subsquently filed sequences listing

SEQUENCE LISTING

<110> Max-Planck-Gesellschaft e.V.

<120> Protein expression and structure solution using
      specific fusion vectors

<130> ST010209-EPA

<140> 01100762.2
<141> 2001-01-12

<160> 3

<170> PatentIn Ver. 2.1

<210> 1
<211> 765
<212> PRT
<213> Artificial Sequence

<220>
<223> Description of Artificial Sequence: Partial myosin
      sequence of Dictyostelium; Component (1) of the
      recombinant protein M761-2R  R238E

<400> 1
Met Asp Gly Thr Glu Asp Pro Ile His Asp Arg Thr Ser Asp Tyr His
  1               5                  10                  15

Lys Tyr Leu Lys Val Lys Gln Gly Asp Ser Asp Leu Phe Lys Leu Thr
             20                  25                  30

Val Ser Asp Lys Arg Tyr Ile Trp Tyr Asn Pro Asp Pro Lys Glu Arg
         35                  40                  45

Asp Ser Tyr Glu Cys Gly Glu Ile Val Ser Glu Thr Ser Asp Ser Phe
     50                  55                  60

Thr Phe Lys Thr Val Asp Gly Gln Asp Arg Gln Val Lys Lys Asp Asp
 65                  70                  75                  80

Ala Asn Gln Arg Asn Pro Ile Lys Phe Asp Gly Val Glu Asp Met Ser
                 85                  90                  95

Glu Leu Ser Tyr Leu Asn Glu Pro Ala Val Phe His Asn Leu Arg Val
            100                 105                 110

Arg Tyr Asn Gln Asp Leu Ile Tyr Thr Tyr Ser Gly Leu Phe Leu Val
        115                 120                 125

Ala Val Asn Pro Phe Lys Arg Ile Pro Ile Tyr Thr Gln Glu Met Val
    130                 135                 140

Asp Ile Phe Lys Gly Arg Arg Arg Asn Glu Val Ala Pro His Ile Phe
145                 150                 155                 160

Ala Ile Ser Asp Val Ala Tyr Arg Ser Met Leu Asp Asp Arg Gln Asn
                165                 170                 175

Gln Ser Leu Leu Ile Thr Gly Glu Ser Gly Ala Gly Lys Thr Glu Asn
            180                 185                 190

Thr Lys Lys Val Ile Gln Tyr Leu Ala Ser Val Ala Gly Arg Asn Gln
        195                 200                 205

Ala Asn Gly Ser Gly Val Leu Glu Gln Gln Ile Leu Gln Ala Asn Pro
    210                 215                 220

Ile Leu Glu Ala Phe Gly Asn Ala Lys Thr Thr Arg Asn Asn Asn Ser
225                 230                 235                 240

Ser Arg Phe Gly Lys Phe Ile Glu Ile Gln Phe Asn Ser Ala Gly Phe
                245                 250                 255

Ile Ser Gly Ala Ser Ile Gln Ser Tyr Leu Leu Glu Lys Ser Arg Val
            260                 265                 270

Val Phe Gln Ser Glu Thr Glu Arg Asn Tyr His Ile Phe Tyr Gln Leu
        275                 280                 285

Leu Ala Gly Ala Thr Ala Glu Glu Lys Lys Ala Leu His Leu Ala Gly
    290                 295                 300

Pro Glu Ser Phe Asn Tyr Leu Asn Gln Ser Gly Cys Val Asp Ile Lys
305                 310                 315                 320

Gly Val Ser Asp Ser Glu Glu Phe Lys Ile Thr Arg Gln Ala Met Asp
                325                 330                 335

Ile Val Gly Phe Ser Gln Glu Glu Gln Met Ser Ile Phe Lys Ile Ile
            340                 345                 350

Ala Gly Ile Leu His Leu Gly Asn Ile Lys Phe Glu Lys Gly Ala Gly
        355                 360                 365

Glu Gly Ala Val Leu Lys Asp Lys Thr Ala Leu Asn Ala Ala Ser Thr
    370                 375                 380

Val Phe Gly Val Asn Pro Ser Val Leu Glu Lys Ala Leu Met Glu Pro
385                 390                 395                 400

Arg Ile Leu Ala Gly Arg Asp Leu Val Ala Gln His Leu Asn Val Glu
                405                 410                 415

Lys Ser Ser Ser Ser Arg Asp Ala Leu Val Lys Ala Leu Tyr Gly Arg
            420                 425                 430

Leu Phe Leu Trp Leu Val Lys Lys Ile Asn Asn Val Leu Cys Gln Glu
        435                 440                 445

Arg Lys Ala Tyr Phe Ile Gly Val Leu Asp Ile Ser Gly Phe Glu Ile
    450                 455                 460

Phe Lys Val Asn Ser Phe Glu Gln Leu Cys Ile Asn Tyr Thr Asn Glu
465                 470                 475                 480

Lys Leu Gln Gln Phe Phe Asn His His Met Phe Lys Leu Glu Gln Glu
                485                 490                 495

Glu Tyr Leu Lys Glu Lys Ile Asn Trp Thr Phe Ile Asp Phe Gly Leu
            500                 505                 510

Asp Ser Gln Ala Thr Ile Asp Leu Ile Asp Gly Arg Gln Pro Pro Gly
        515                 520                 525

Ile Leu Ala Leu Leu Asp Glu Gln Ser Val Phe Pro Asn Ala Thr Asp
    530                 535                 540

Asn Thr Leu Ile Thr Lys Leu His Ser His Phe Ser Lys Lys Asn Ala
545                 550                 555                 560

Lys Tyr Glu Glu Pro Arg Phe Ser Lys Thr Glu Phe Gly Val Thr His
                565                 570                 575

Tyr Ala Gly Gln Val Met Tyr Glu Ile Gln Asp Trp Leu Glu Lys Asn
            580                 585                 590

Lys Asp Pro Leu Gln Gln Asp Leu Glu Leu Cys Phe Lys Asp Ser Ser
        595                 600                 605

Asp Asn Val Val Thr Lys Leu Phe Asn Asp Pro Asn Ile Ala Ser Arg
    610                 615                 620

Ala Lys Lys Gly Ala Asn Phe Ile Thr Val Ala Ala Gln Tyr Lys Glu
625                 630                 635                 640

Gln Leu Ala Ser Leu Met Ala Thr Leu Glu Thr Thr Asn Pro His Phe
                645                 650                 655

Val Arg Cys Ile Ile Pro Asn Asn Lys Gln Leu Pro Ala Lys Leu Glu
            660                 665                 670

Asp Lys Val Val Leu Asp Gln Leu Arg Cys Asn Gly Val Leu Glu Gly
        675                 680                 685

Ile Arg Ile Thr Arg Lys Gly Phe Pro Asn Arg Ile Ile Tyr Ala Asp
    690                 695                 700

Phe Val Lys Arg Tyr Tyr Leu Leu Ala Pro Asn Val Pro Arg Asp Ala
705                 710                 715                 720

Glu Asp Ser Gln Lys Ala Thr Asp Ala Val Leu Lys His Leu Asn Ile
                725                 730                 735

Asp Pro Glu Gln Tyr Arg Phe Gly Ile Thr Lys Ile Phe Phe Arg Ala
            740                 745                 750

Gly Gln Leu Ala Arg Ile Glu Glu Ala Arg Glu Gln Arg
        755                 760                 765

<210> 2
<211> 1016
<212> PRT
<213> Artificial Sequence

<220>
<223> Description of Artificial Sequence: Whole sequence
      of recombinant protein M761-2R R238 E

<220>
<223> The protein comprises as component (1) the aa
      sequence of myosin II motor domain of
      Dictyostelium, a three aa linker region and the
      a-actinin aa sequence

<400> 2
Met Asp Gly Thr Glu Asp Pro Ile His Asp Arg Thr Ser Asp Tyr His
  1               5                  10                  15

Lys Tyr Leu Lys Val Lys Gln Gly Asp Ser Asp Leu Phe Lys Leu Thr

20                  25                  30

Val Ser Asp Lys Arg Tyr Ile Trp Tyr Asn Pro Asp Pro Lys Glu Arg
        35                  40                  45

Asp Ser Tyr Glu Cys Gly Glu Ile Val Ser Glu Thr Ser Asp Ser Phe
    50                  55                  60

Thr Phe Lys Thr Val Asp Gly Gln Asp Arg Gln Val Lys Lys Asp Asp
65                  70                  75                  80

Ala Asn Gln Arg Asn Pro Ile Lys Phe Asp Gly Val Glu Asp Met Ser
                85                  90                  95

Glu Leu Ser Tyr Leu Asn Glu Pro Ala Val Phe His Asn Leu Arg Val
            100                 105                 110

Arg Tyr Asn Gln Asp Leu Ile Tyr Thr Tyr Ser Gly Leu Phe Leu Val
        115                 120                 125

Ala Val Asn Pro Phe Lys Arg Ile Pro Ile Tyr Thr Gln Glu Met Val
    130                 135                 140

Asp Ile Phe Lys Gly Arg Arg Arg Asn Glu Val Ala Pro His Ile Phe
145                 150                 155                 160

Ala Ile Ser Asp Val Ala Tyr Arg Ser Met Leu Asp Asp Arg Gln Asn
                165                 170                 175

Gln Ser Leu Leu Ile Thr Gly Glu Ser Gly Ala Gly Lys Thr Glu Asn
            180                 185                 190

Thr Lys Lys Val Ile Gln Tyr Leu Ala Ser Val Ala Gly Arg Asn Gln
        195                 200                 205

Ala Asn Gly Ser Gly Val Leu Glu Gln Gln Ile Leu Gln Ala Asn Pro
    210                 215                 220

Ile Leu Glu Ala Phe Gly Asn Ala Lys Thr Thr Arg Asn Asn Asn Ser
225                 230                 235                 240

Ser Arg Phe Gly Lys Phe Ile Glu Ile Gln Phe Asn Ser Ala Gly Phe
                245                 250                 255

Ile Ser Gly Ala Ser Ile Gln Ser Tyr Leu Leu Glu Lys Ser Arg Val
            260                 265                 270

Val Phe Gln Ser Glu Thr Glu Arg Asn Tyr His Ile Phe Tyr Gln Leu

        275                 280                 285

Leu Ala Gly Ala Thr Ala Glu Glu Lys Lys Ala Leu His Leu Ala Gly
    290                 295                 300

Pro Glu Ser Phe Asn Tyr Leu Asn Gln Ser Gly Cys Val Asp Ile Lys
305                 310                 315                 320

Gly Val Ser Asp Ser Glu Glu Phe Lys Ile Thr Arg Gln Ala Met Asp
                325                 330                 335

Ile Val Gly Phe Ser Gln Glu Glu Gln Met Ser Ile Phe Lys Ile Ile
            340                 345                 350

Ala Gly Ile Leu His Leu Gly Asn Ile Lys Phe Glu Lys Gly Ala Gly
        355                 360                 365

Glu Gly Ala Val Leu Lys Asp Lys Thr Ala Leu Asn Ala Ala Ser Thr
    370                 375                 380

Val Phe Gly Val Asn Pro Ser Val Leu Glu Lys Ala Leu Met Glu Pro
385                 390                 395                 400

Arg Ile Leu Ala Gly Arg Asp Leu Val Ala Gln His Leu Asn Val Glu
                405                 410                 415

Lys Ser Ser Ser Ser Arg Asp Ala Leu Val Lys Ala Leu Tyr Gly Arg
            420                 425                 430

Leu Phe Leu Trp Leu Val Lys Lys Ile Asn Asn Val Leu Cys Gln Glu
        435                 440                 445

Arg Lys Ala Tyr Phe Ile Gly Val Leu Asp Ile Ser Gly Phe Glu Ile
    450                 455                 460

Phe Lys Val Asn Ser Phe Glu Gln Leu Cys Ile Asn Tyr Thr Asn Glu
465                 470                 475                 480

Lys Leu Gln Gln Phe Phe Asn His His Met Phe Lys Leu Glu Gln Glu
                485                 490                 495

Glu Tyr Leu Lys Glu Lys Ile Asn Trp Thr Phe Ile Asp Phe Gly Leu
            500                 505                 510

Asp Ser Gln Ala Thr Ile Asp Leu Ile Asp Gly Arg Gln Pro Pro Gly
        515                 520                 525

Ile Leu Ala Leu Leu Asp Glu Gln Ser Val Phe Pro Asn Ala Thr Asp

    530                 535                 540

Asn Thr Leu Ile Thr Lys Leu His Ser His Phe Ser Lys Lys Asn Ala
545                 550                 555                 560

Lys Tyr Glu Glu Pro Arg Phe Ser Lys Thr Glu Phe Gly Val Thr His
                565                 570                 575

Tyr Ala Gly Gln Val Met Tyr Glu Ile Gln Asp Trp Leu Glu Lys Asn
            580                 585                 590

Lys Asp Pro Leu Gln Gln Asp Leu Glu Leu Cys Phe Lys Asp Ser Ser
        595                 600                 605

Asp Asn Val Val Thr Lys Leu Phe Asn Asp Pro Asn Ile Ala Ser Arg
    610                 615                 620

Ala Lys Lys Gly Ala Asn Phe Ile Thr Val Ala Ala Gln Tyr Lys Glu
625                 630                 635                 640

Gln Leu Ala Ser Leu Met Ala Thr Leu Glu Thr Thr Asn Pro His Phe
                645                 650                 655

Val Arg Cys Ile Ile Pro Asn Asn Lys Gln Leu Pro Ala Lys Leu Glu
            660                 665                 670

Asp Lys Val Val Leu Asp Gln Leu Arg Cys Asn Gly Val Leu Glu Gly
        675                 680                 685

Ile Arg Ile Thr Arg Lys Gly Phe Pro Asn Arg Ile Ile Tyr Ala Asp
    690                 695                 700

Phe Val Lys Arg Tyr Tyr Leu Leu Ala Pro Asn Val Pro Arg Asp Ala
705                 710                 715                 720

Glu Asp Ser Gln Lys Ala Thr Asp Ala Val Leu Lys His Leu Asn Ile
                725                 730                 735

Asp Pro Glu Gln Tyr Arg Phe Gly Ile Thr Lys Ile Phe Phe Arg Ala
            740                 745                 750

Gly Gln Leu Ala Arg Ile Glu Glu Ala Arg Glu Gln Arg Leu Gly Ser
        755                 760                 765

Glu Gln Thr Lys Ser Asp Tyr Leu Lys Arg Ala Asn Glu Leu Val Gln
    770                 775                 780

Trp Ile Asn Asp Lys Gln Ala Ser Leu Glu Ser Arg Asp Phe Gly Asp

785                 790                 795                 800

Ser Ile Glu Ser Val Gln Ser Phe Met Asn Ala His Lys Glu Tyr Lys
                805                 810                 815

Lys Thr Glu Lys Pro Pro Lys Gly Gln Glu Val Ser Glu Leu Glu Ala
            820                 825                 830

Ile Tyr Asn Ser Leu Gln Thr Lys Leu Arg Leu Ile Lys Arg Glu Pro
        835                 840                 845

Phe Val Ala Pro Ala Gly Leu Thr Pro Asn Glu Ile Asp Ser Thr Trp
    850                 855                 860

Ser Ala Leu Glu Lys Ala Glu Gln Glu His Ala Glu Ala Leu Arg Ile
865                 870                 875                 880

Glu Leu Lys Arg Gln Lys Lys Ile Ala Val Leu Leu Gln Lys Tyr Asn
                885                 890                 895

Arg Ile Leu Lys Lys Leu Glu Asn Trp Ala Thr Thr Lys Ser Val Tyr
            900                 905                 910

Leu Gly Ser Asn Glu Thr Gly Asp Ser Ile Thr Ala Val Gln Ala Lys
        915                 920                 925

Leu Lys Asn Leu Glu Ala Phe Asp Gly Glu Cys Gln Ser Leu Glu Gly
    930                 935                 940

Gln Ser Asn Ser Asp Leu Leu Ser Ile Leu Ala Gln Leu Thr Glu Leu
945                 950                 955                 960

Asn Tyr Asn Gly Val Pro Glu Leu Thr Glu Arg Lys Asp Thr Phe Phe
                965                 970                 975

Ala Gln Gln Trp Thr Gly Val Lys Ser Ser Ala Glu Thr Tyr Lys Asn
            980                 985                 990

Thr Leu Leu Ala Glu Leu Glu Arg Leu Gln Lys Ile Glu Asp Ala Leu
        995                 1000                1005

His His His His His His His His
    1010                1015

<210> 3
<211> 3048
<212> DNA

<213> Artificial Sequence

<220>
<223> Description of Artificial Sequence: DNA sequence
      coding for recombinant protein M761-2R R238E

<400> 3
atggatggta ccgaggatcc aattcatgat agaacttcag attatcacaa atacttaaaa 60
gttaaacaag gtgattctga tttatttaaa cttactgttt cagataagag atacatttgg 120
tataatccag atccaaaaga aagagattca tatgaatgtg gtgaaattgt ttcagaaacc 180
tctgattctt tcacattcaa aaccgttgat ggtcaagaca gacaagtcaa aaaggatgat 240
gccaatcaac gtaatccaat caaattcgat ggtgtcgaag atatgtctga attatcatac 300
ctcaatgaac cagcagtttt ccacaatctc cgtgttcgtt acaatcaaga tttaatttac 360
acctattcag gtctcttttt ggttgccgtc aatccattca agagaattcc aatctacact 420
caagagatgg ttgatatctt caaaggtcgt agaagaaatg aagttgcccc acatattttc 480
gccatttctg atgttgccta tcgttcaatg ttagatgatc gtcaaaatca atcactctta 540
atcactggtg aatctggtgc tggtaagact gaaaacacca aaaaggtcat tcaatatctt 600
gcatctgtcg ctggtcgtaa tcaagccaat ggtagtggtg tattggaaca acaaattctc 660
caagccaatc caatccttga agcttttggt aatgccaaaa ccacccgtaa caacaattca 720
tctcgtttcg gtaaattcat tgaaattcaa ttcaacagtg ctggtttcat tagtggtgct 780
tcaattcaat cctacctttt agagaaatca cgtgtcgttt tccaatctga aaccgaacgt 840
aattatcaca ttttctatca actcttagct ggtgccaccg ccgaagaaaa gaaagctctt 900
cacttggctg gtccagaatc attcaactac ttaaatcaaa gtggttgtgt tgatatcaaa 960
ggtgtctctg atagtgaaga attcaaaatc actcgtcaag ctatggacat tgttggtttc 1020
tcacaagaag aacaaatgtc aatctttaag atcattgctg gtatcttaca tttaggtaac 1080
atcaaattcg aaaaaggtgc tggtgaaggt gctgtcctca aagacaaaac cgccctcaac 1140
gctgcttcaa ccgtctttgg tgtcaatcca tcagtccttg aaaaggctct catggaacca 1200
cgtattttag ccggtcgtga tttagttgct caacatctca acgttgaaaa atcctcatca 1260
tcaagagacg ctcttgtcaa agctctctat ggtcgtcttt tcctctggtt ggtcaaaaag 1320
atcaacaatg tcctctgtca agagagaaaa gcttacttta ttggtgtttt ggatatttca 1380
ggttttgaaa ttttcaaagt caattcattc gaacaattat gtatcaatta taccaatgaa 1440
aaactccaac aattcttcaa tcaccatatg ttcaaattgg aacaagaaga atatcttaaa 1500
gagaaaatca attggacttt catcgatttt ggtcttgatt cacaagccac tatcgattta 1560
attgatggtc gtcaaccacc aggtatttta gctcttttgg atgaacaatc tgttttccca 1620
aatgccaccg ataatacttt aatcaccaaa ctccacagtc actttagcaa gaagaacgcc 1680
aaatacgaag aaccacgttt ctccaaaacc gaatttggtg ttacccatta tgctggtcaa 1740
gtcatgtatg agattcaaga ttggttagaa aagaacaaag atccattaca acaagatctc 1800
gaactttgct tcaaagattc atcagacaac gttgtcacca aacttttcaa tgatccaaac 1860
attgccagtc gtgcaaagaa aggtgcaaac tttatcactg tcgccgctca atacaaggaa 1920
caattagcct cactcatggc nacccttgaa accaccaacc cacatttcgt tcgttgtatc 1980
attccaaaca acaaacaatt accagccaaa ctcgaagata aagttgtcct cgaccaatta 2040
cgttgcaatg gtgtcctcga aggtattcgt attactcgta aaggtttccc aaatcgtatt 2100
atctatgccg atttcgtcaa acgttactat ttattagctc caaacgttcc aagagacgct 2160
gaagactcac aaaaagccac cgatgctgtt ctcaaacatc ttaacattga tccagaacaa 2220
tatcgtttcg gtatcaccaa gattttcttc cgtgccggtc aattagctcg tattgaagaa 2280
gctcgtgaac aacgtctagg atccgaacaa accaaatctg attatcttaa aagagccaat 2340
gaactcgttc aatggattaa cgataaacaa gcatcacttg aatcacgtga ttttggtgat 2400
tccatcgaat ctgttcaaag tttcatgaac gctcataaag aatataaaaa aaccgaaaaa 2460

ccaccaaagg gtcaagaagt ctctgaattg gaagctatct acaattcatt acaaactaaa 2520
ttacgtttaa ttaaacgtga accatttgtt gcaccagctg gtctcactcc aaatgaaatc 2580
gattccactt ggtccgcttt agagaaagct gaacaagaac atgctgaagc cctccgtatt 2640
gaactcaaac gtcaaaagaa aattgcagtt ctcttacaaa aatacaatcg tattctcaag 2700
aaactcgaaa actgggccac caccaaatct gtctacctcg gttccaatga aaccggtgac 2760
agtatcactg ctgttcaagc taaattaaag aatttagaag cttttgatgg tgaatgtcaa 2820
tcattggaag gtcaatcaaa ctctgatctc ctcagcattc ttgctcaatt aactgaactc 2880
aactacaatg gtgtaccaga actcactgaa cgtaaagata cattctttgc tcaacaatgg 2940
actggtgtta aatcatctgc tgaaacctac aaaaacactc ttttagctga acttgaaaga 3000
ctccaaaaga ttgaagatgc attacatcat catcatcatc atcaccac 3048

## Description

The present invention relates to a recombinant protein being composed of three components (1), (2) and (3), a DNA sequence encoding such a recombinant protein, a vector expressing such a recombinant protein, a host cell transformed with a vector, a method for producing a recombinant protein, and methods for purification, crystallization and structure elucidation of the recombinant protein.

The first step, and perhaps the single most important step, in the crystallization of a macromolecule, e.g. a protein, is its purification. Any impurities of the protein solution to be used for crystallization may impair crystal quality or, even worse, preclude the formation of crystals at all.

Procedures for accomplishing the highest degree of purification possible have been under development for more than 200 years, and recent times have seen an explosion in the invention of new methods and refinement of old. There is a variety of methods that exemplify how problems of protein purification for protein analysis or protein crystallization have been approached.

One such method is fractionation with salts and other precipitants. Hereby, proteins are precipitated from a complex mixture (e.g. a physiological fluid) by addition of various concentrations of different salts. Because individual proteins precipitate at different salt concentrations, this "salting out" phenomenon provided a method for selectively precipitating, and thereby purifying, unique proteins from a mixture (Morris and Morris, 1964, Separation methods in biochemistry, Pitman, London, GB). A minor disadvantage of salt fractionation is that protein preparations, be they supernatants or precipitates, are left with high residuals of salt. This may seriously interfere with the evaluation of activity and purity and with subsequent purification procedures. The most common of these methods is dialysis in celluloid or collodian tubes.

Apart from varying the concentration of a salt, proteins may be selectively precipitated and fractionated by the addition of a variety of organic solvents (Cohn et al., 1947, Crystallization of serum albumin from ethanol/water mixtures, J. Am. Chem. Soc. 69, 1753). This is generally carried out at subzero temperatures ranging to -30°C to enhance the precipitation effect and to minimize the denaturation of the protein. In addition to salt and organic solvents, other materials have been used to precipitate and fractionate a mixture of proteins. Some of these materials are, for example, protamine (a mixture of small basic proteins) and polyeneimine (a basic organic polymer), which apparently cross-links some protein via electrostatic bridges. Moreover, metal ions or organic polymers, such as polyethylene glycol (PEG), were extensively used for purification purposes. PEG seems to act as a hybrid between an alcohol and a salt and their precise properties may vary as a function of mean polymer length.

Still another method of protein purification is the selection of proteins with heat or pH. pH is effective because most proteins exhibit pH-dependent solubility minima and precipitate or even crystallize from solution at particular values, whereas the property of protein heat stability may sometimes provide a valuable purification step.

Other protein purification methods based on physical techniques are also well-known to a person skilled in the art. E.g. centrifugation has to be mentioned, whereby a solution containing multiple components varying in weight, size, and density is deployed in a tube and rotated at high angular velocity. An almost preparative centrifugation is conducted on some gradient with various density from the top to the bottom of the centrifuge tube. Two common techniques utilized in connection with density gradient separation are sedimentation velocity and sedimentation equilibrium or isopycnic centrifugation. Furthermore, electrophoretic separation methods (Svensson, 1947, Preparative electrophoresis and ionophoresis, Adv. Protein Chem. 4:251) are routinely used and are based on the application of an electrical field across and insoluble, porous support medium permeated by a buffer solution. Dependent on the net charge of the proteins to be separated they will experience and electromotive force and migrate toward one electrode (cathode or anode). For the separation of proteins, polyacrylamide gels as support medium have shown to have almost ideal properties.

Finally, chromatographic methods are especially well suited to separate proteins and to purify the target protein for later crystallization steps. Classic ion exchange chromatography is simply conducted by packing a vertical hollow glass column with an insoluble resin or colloidal matrix that exhibits an array of positively charged (anion exchange chromatography) or negatively charged chemical groups (cation exchange chromatography). Ion exchange chromatography is based on the fact that a positively charged protein will be retarded or bound to electrostatic interactions with a matrix carrying negatively charged groups or vice-versa for negatively charged proteins. Dependent on their respective net charge the proteins to be separated will appear in the eluent sequentially with time (or volume). Molecules tightly bound to the matrix may be eluted from the column by competition with other charged ions. In contrast to ion exchange chromatography, molecular sieve chromatography (also called gel permeation chromatography) separates molecules on the basis of molecular weight and shape. Hereby, macromolecules, like proteins, are induced to flow by gravity or pressure through a column containing a matrix of microscopic beads perforated with a vast network of channels. Thereby, the high molecular sieving effect will influence the speed in passing from the top to the bottom of the column leading to the inverse effect that larger molecules will appear first in the column eluent. Finally, absorption chromatography, HPLC (high performance liquid chromatography), and affinity chromatography are also well established as biochemical purification methods.

All of the above-mentioned methods exhibit certain advantages and disadvantages. Consequently, the person skilled in the art will choose the purification method which appears to be most appropriate for a given system.

Since a number of years purification methods take advantage of recombinant proteins (Kane and Hartley, 1988, Development of expression systems for production of high levels of protein, Trends Biotechnol. 6: 95). Recombinant proteins are produced by recombinant DNA techniques in bacteria, yeast or other organisms such as virus infected mammalian or insect cells. The advantage of recombinant proteins is based on genetically designed elements, that aid the biochemist in applying one of the aforementioned physical or biochemical purification methods. For example, a series of histidine residues, a so-called "his-tag", may be appended to the carboxyl terminus of a recombinant protein. Such a histidin-appendix makes it easier to isolate the expressed protein on a copper or nickel containing chromatographic resin, the latter being available commercially in prepacked columns.

A second procedure in wide use for the purification of recombinant proteins is the fusion of an expressed protein with the enzyme glutathione sulfur transferase (GST). This enzyme has a very high affinity for the small peptide glutathione. Following expression of the protein, an extract of the cells is passed over a small chromatography column containing a matrix conjugated with glutathione. The chimeric protein is then reversibly bound on the column through the GST, contaminants are washed from the column, and finally the recombinant protein is eluted with free glutathione and collected. The GST may then be cleaved from the chimer by a specific protease to produce the free recombinant protein. Again, the chromatographic matrix may be obtained commercially in prepacked columns.

Furthermore, e.g. pMAL™ (by New England Biolabs Inc.) is used as protein fusion and purification system as prior art. This system comprises the insertion of the cloned gene into a pMAL vector downstream from the malE gene, which encodes maltose binding protein (MBP). The fusion protein (target protein and MBP) is expressed in large quantities and purified by affinity chromatography for MBP using amylose resin. Finally, MBP is cleaved from the target protein by a specific protease.

These techniques utilizing recombinant proteins allow to obtain extraordinarily pure fractions of the target protein. However, advantageous conditions for purification, crystallization and structural analysis have to be tested using the MBP/target protein or GST/target protein fusion systems for each single recombinant and/or target protein. Particularly, (i) there are still complex chromatographic (in vitro) purification steps required for obtaining pure fractions of the target protein and (ii) further steps of analysis, like crystallization or structure determination, are complicated by the unknown properties of the target protein, like e.g. the crystallization conditions of a specific target protein purified as MBP or GST fusion protein.

The object of the present invention is to overcome the above-mentioned disadvantages of the prior art, particularly to provide a system which allows to reduce considerably the time effort for purification and subsequent crystallization as well as structure determination of any protein to be analysed.

This object is achieved by a recombinant protein according to claim 1, a DNA sequence encoding such a protein according to claim 8, a vector according to claim 10, a transformed host cell according to claim 12, a method for producing a recombinant protein according to claim 14, and methods for purification, crystallization and structure elucidation according to claims 15, 21 and 23.

Therefore, it is an object of the present invention, in one aspect, to provide recombinant proteins which contain as component (1) an amino acid sequence of a member of the myosin or kinesin protein superfamilies or an amino acid sequence of an analog, fragment or derivative of a member of the myosin or kinesin protein superfamilies, as component (2) any amino acid sequence of at least 20 amino acids in length (target protein sequence), and as component (3) a linker region of at least 2 amino acids between components (1) and (2) (claim 1). However, the subject-matter of the present invention works with any protein or fragment, derivative or analog thereof as component (1), which binds to a any molecule or structure of the cytoskeleton or a cell membrane in a ligand dependent manner. Particularly preferred as component (1) are molecules, which exhibit a flexible region, particularly at the C-terminal region of component (1), in order to sample for multiple conformations.

As mentioned above, the present invention further concerns analogs, fragments and derivatives of the recombinant protein of the invention. The preparation of such analogs, fragments and derivatives is by a standard procedure (Sambrook et al. (1989), Molecular Cloning: A Laboratory Manual, Cold Spring Harbor, New York) in which in the DNA sequences encoding the inventive recombinant protein, one or more codons may be deleted, added or substituted by another, to yield analogs having at least one amino acid residue change with respect to the native recombinant protein, particularly with respect to the native amino acid sequence of component (1) or of component (2) of the recombinant protein of the invention.

Analogs that substantially correspond to the native sequence of one or more components of the inventive recombinant protein are those polypeptides, in which one or more amino acids of the native protein's amino acid sequence has/have been replaced by another amino acid, deleted and/or inserted. In a preferred embodiment of the present invention, the resulting components ((1) or (2)) being incorporated into the recombinant protein of the invention exhibit substantially the same or even higher biological activity as the corresponding native protein to which it corresponds or exhibit at least structurally similar properties as the native protein to which the component corresponds. In order to substantially correspond to the native sequence of component (1) or (2) of the recombinant protein of the invention, the changes in the sequence of the components are generally and preferably relatively minor, such as isoforms. Although the number of changes may be more than 10, preferably there are no more than 10 changes, more preferable no more than 5 and most preferably no more than 3 changes in component (1) or (2) as compared to the respective native sequence. While any technique may be used to find potentially biologically active sequences of a component of the inventive recombinant protein, which substantially correspond to the respective native proteins, one such technique is the use of conventional mutagenesis techniques on the DNA encoding the protein, resulting in a few modifications. The sequences used for component (1) or (2) in the recombinant protein of the invention which are expressed by such clones, may then be screened for their ability e.g. to bind to their native binding partners, mediate activity etc. , in other words fulfil their biological role.

Conservative "changes" are those changes which would not be expected to change the activity of the protein and are usually the first to be screened as these would not be expected to substantially change size, charge or structure of the polypeptide sequence used as component in the recombinant protein of the invention and thus would not be expected to change the biological properties of the corresponding native sequence. For example, conservative substitutions are assumed, if (a) small aliphatic, non-polar or slightly polar residues are substituted by other residues belonging to the same group, (b) polar negatively charged residues and their amides are exchanged for other residues belonging to the same group, (c) polar positively charged residues are exchanged for polar positively residues, (d) large aliphatic non-polar residues are exchanged for large aliphatic non-polar residues, and (e) finally, aromatic residues are substituted by other aromatic residues. In most cases, analogs being used as component (1) or (2) of the recombinant protein of the invention are defined (according to the present invention) as sequences with substitutions which do not produce radical changes in the characteristics of the corresponding native protein or polypeptide molecule. Characteristics may be the specific secondary structure of a sequence, e.g. α-helix or β-sheet, as well as its specific biological activity.

It is noted that apart from sequences being used as component (1) or (2) for a recombinant protein according to the present invention, which are based on conservative substitutions as discussed above, analogs with more random changes, which lead to a radical or more radical change in biological activity or structure of the analog as compared to the native sequence are also within the scope of the present invention.

At the genetic level, these analogs are generally prepared by site-directed mutagenesis of nucleotides in the DNA encoding the inventive recombinant protein or the component of the recombinant protein, respectively, thereby producing DNA encoding the analog and thereafter synthesizing the DNA and expressing the polypeptide in recombinant cell culture. Insofar, it is referred to Ausübel et al., Current Protocols in Molecular Biology, Green Publications and Wiley Intersigns, New York, New York, 1987 - 1995; Sambrook et al., Molecular Cloning: Laboratory Manual, Cold Spring Harbor Laboratory, New York, 1989, the disclosure of which is incorporated herein by reference. Furthermore, site-specific mutagenesis allows the production of analogs through the use of specific oligonucleotide sequences that encode the DNA sequence of the desired mutation. The technique of site-directed mutagenesis is exemplified by publications such as Adelman et al., DNA 2: 183 (1983)), the disclosure of which is incorporated herein by reference. Typical vectors useful in site-directed mutagenesis include vectors such as M13-phage, for example as disclosed by Messing et al. (3^{rd} Cleveland Symposium on Macromolecules and recombinant DNA, editor A. Walton, Elsevier, Amsterdam (1981)), the disclosure of which is incorporated herein by reference.

As far as derivatives of the native sequence of components of the recombinant protein of the present invention are concerned, derivatives may be prepared by standard modifications of the side groups of one or more amino acid residues of the recombinant protein of the invention, its analogs or fragments or by conjugation of the native sequence used as component (1) or (2) of the inventive recombinant protein, its analogs or fragments, to another molecule, e.g. an antibody, enzyme, receptor, etc. Accordingly, "derivatives" as used herein cover derivatives which may be prepared from the functional groups which occur as side chains on the residues or from the N- or C- terminal groups by means known in the art. Derivatives may have chemical moieties such as carbohydrates or phosphate residues. For example, derivatives may include aliphatic esters of the carboxyl groups, amides of the carboxyl group by reaction with ammonia or with primary or secondary amines, N-acyl derivatives or free amino groups of the amino acid residues formed with acyl moieties or O-acyl derivatives of free hydroxyl groups (for example of seryl or threonyl residues) formed with acyl moieties. The term derivative includes - generally spoken - all polypeptide sequences for one component (1 and/or 2) of the recombinant protein sequence which are larger in sequence than the corresponding native sequence. The addition of at least one, typically more than 10 amino acids may take place intrasequentially or at the N- or C-terminus of the sequence of component (1) and/or (2) of an inventive recombinant protein. In a preferred embodiment of the present invention, additional amino acids are appended to the N-terminus of component (1) or the C-terminus of component (2) coinciding with the N-terminus and the C-terminus of the inventive recombinant protein. In another preferred embodiment, additional amino acid sequences are inserted intrasequentially, preferably in such a way that the secondary and/or tertiary structure is not destroyed. Typically these insertions are placed at the surface of the protein, e.g. in β-bends. Preferably, one or more S-containing residues (particularly Cys) are inserted or other residues with a potential for binding heavy metal atoms (e.g. Hg-ions). The introduction of additional heavy metal binding residues at sites on the surface of the recombinant protein of the invention may be accompanied by substitution and/or deletion of native heavy metal binding residues in order create novel heavy metal atom binding sites. Such a procedure is particularly suitable for gaining additional phasing information for structure determination of large protein complexes by X-ray crystallography.

In a non-limiting manner, "tag"-sequences may be contained in the recombinant protein and, particularly, may be added to the N- or C-terminus of the recombinant protein of the invention (claim 7). These "tag"-sequences typically have antigenic character for commercially available antibodies, e.g. an N-terminal "Flag-tag" having the sequence DYKDDDDK (one-letter-code). Other suitable "tag"-sequences are, for example, N- or C-terminal polyhistidine tags.

Furthermore, component (1) and/or (2) as parts of the recombinant protein of the invention may be fusion proteins. Particularly preferred are sequences fused to the N-terminus of the native sequence of component (1) or to the N-terminus of an analog, derivative or fragment thereof. For example, component (1) of the recombinant protein may be fused N-terminally to a marker protein, e.g. an enzyme marker or a fluorescence marker, such as GFP (green fluorescence protein), or any sequence being suitable as epitope for an antibody or even to an antibody or an antibody fragment itself.

Finally, "fragments" of the native sequence of any protein being used as component (1) or (2) of the recombinant protein according to the present invention may be used, e.g. fragments of proteins of the myosin or kinesin protein superfamilies, particularly fragments being deleted C-terminally, the deletion comprising at least ten, and more preferably at least 50 amino acids. However, the fragment of the native sequence may also contain deletions at the N- and/or C- terminus and/or intrasequentially in component (1) and/or component (2) of a recombinant protein of the invention. In a preferred embodiment, component (1) consists of a fragment comprising the catalytic domain of a member of the myosin or kinesin protein superfamilies of any eukaryotic organism. In other words, component (1) corresponds preferably to a fragment containing the myosin or kinesin motor domain. Within the scope of the present invention are therefore recombinant proteins characterized in that they contain as component (1) an amino acid sequence for the motor domain of a kinesin or myosin family member or an analog, fragment or derivative thereof (claim 3).

In a preferred embodiment of the present invention, the recombinant protein according to the present invention contains as component (1) an amino acid sequence of a member of the myosin I, II, III, IV, V, VIII, VI, X, or XI or a member of kinesin I or II families or an amino acid sequence of an analog, fragment or derivative of a member of the aforementioned myosin and kinesin families (claim 2). Preferably, component (1) contains a member of the myosin II family of any eukaryotic organism or an analog, fragment or derivative thereof. Still further preferred, component (1) contains myosin II of *Dictyostelium* or an analog, fragment or derivative thereof. Further preferred embodiments of the present invention for component (1) are proteins containing the motor domains of smooth muscle myosin II (e.g. chicken gizzard myosin), vertebrate or amoeboid forms of myosin I (bovine brushborder myosin), *Dictyostelium* myoID, vertebrate myosin V , myosin VI, Toxoplasma gondii (e.g. TgMyoA) and Plasmodium sp. myosin XIV, vertebrate kinesin (human kinesin I), amoeboid or fungal kinesins (e.g. *Dictyostelium* kinesin 7).

Preferably, a recombinant protein according to the present invention contains as linker component (3) a stretch of at least 3 amino acids, more preferably 5 and still further preferably 10 amino acids. Particularly preferred is a linker sequence, which contains a protease cleavage site. A recognition sequence for any protease may be used, for example, the cleavage site may contain the recognition sequence for factor Xa, thrombin or for the protease TEV (recognition sequence: ENLYFQG) or the Soldati protease. However, it is within the scope of this invention that component (1) and (2) are directly fused together without insertion of a linker sequence.

If linker component (3) consists of three amino acids, it is preferred to chose a sequence with at least one Gly residue, particularly in the second position of the linker stretch (claim 4). Still further preferred is a linker with the following sequence: N-Leu-Gly-Arg-C.

Recombinant protein according to any of the preceding claims characterized in that it contains as component (2) (target protein) the sequence of an esterase, hydrolase, phosphatase, kinase, protease, channel, structural protein (e.g. coronin, spectrin), receptor, particularly a neuronal or immunologically relevant receptor (e.g. superfamily of TNF receptors), transcription factor, DNA/RNA-binding protein, lipoprotein, glycoprotein or an analog, derivative or fragment thereof (claim 5).

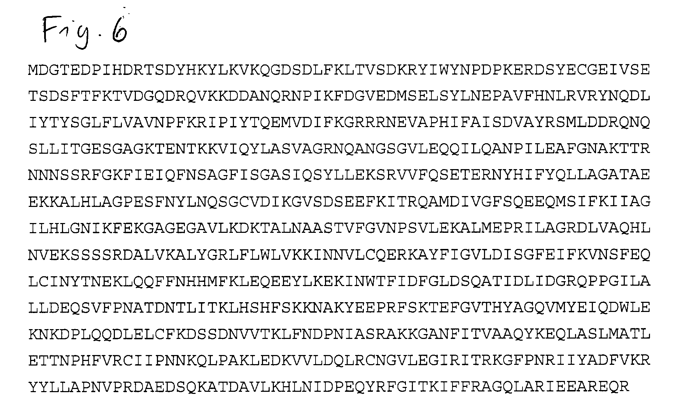

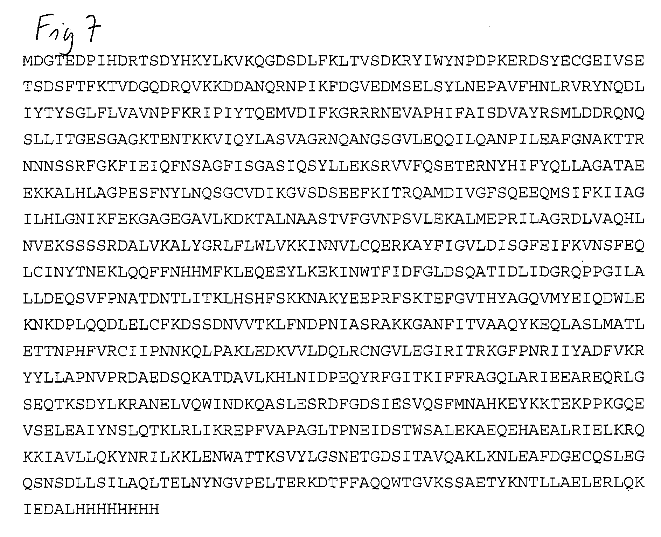

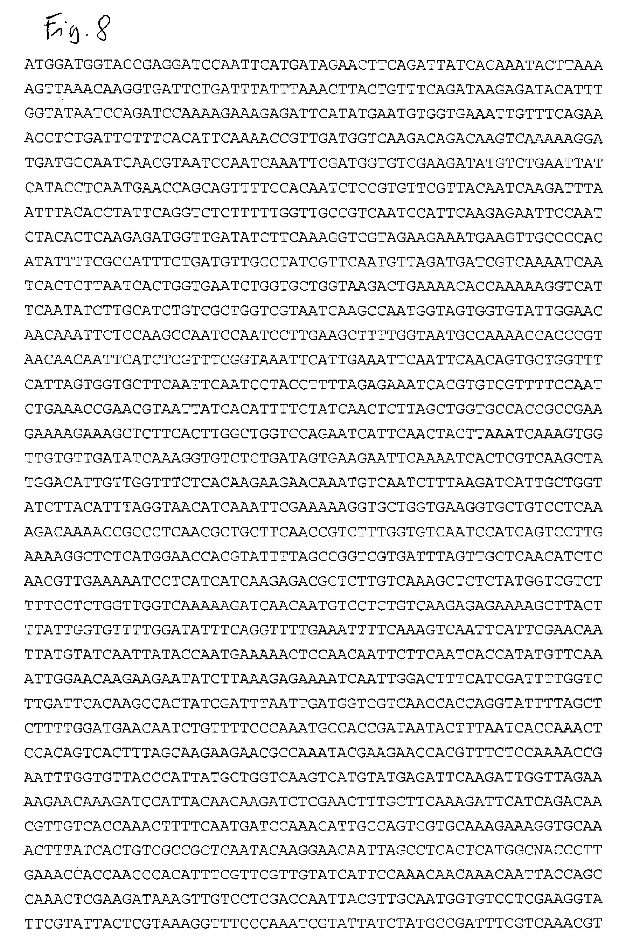

Particularly, a recombinant protein according to the present invention has as component (1) an amino acid sequence as exhibited in figure 6 or an analog, derivative and/or fragment thereof. It is preferred to combine the sequence of figure 6 with a linker sequence (3) containing a protease recognition site as exemplified above or amino acid sequence Leu-Gly-Arg (claim 6). Still further preferred is a recombinant protein having a sequence as shown in fig. 7.

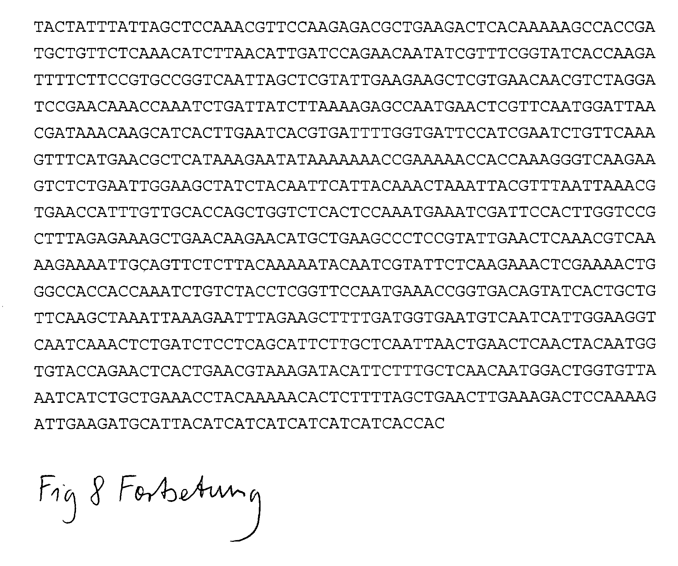

A second aspect of the present invention relates to a DNA sequence which contains a sequence which codes for an amino acid sequence (for a recombinant protein) according to the present invention (claim 8). In particular, the present invention provides a DNA sequence selected from the group consisting of: (a) a cDNA sequence derived from the coding region of a recombinant protein according to the present invention; (b) DNA sequences capable of hybridization to a sequence of (a) under moderately stringent conditions; and (c) DNA sequences which are degenerate as a result of the genetic code to the DNA sequences defined in (a) and (b). Another specific embodiment of the above DNA sequence of the invention is a DNA sequence comprising at least part of a sequence encoding for a recombinant protein as depicted in fig. 8, particularly the segment of fig. 8 which codes for the myosin motor domain. Nucleic acid stretches encoding for a recombinant protein of the present invention may be detected, obtained and/or modified, in vitro, in-situ and/or in vivo, by the use of known DNA or RNA amplification techniques, such as PCR and chemical oligonucleotide synthesis. PCR allows for the amplification (increase in number) of a specific DNA sequence by repeated DNA polymerase reactions. This reaction may be used as a replacement for cloning. All that is required is a knowledge of the nucleic acid sequence. In order to carry out PCR, primers are designed which are complementary to the sequence of interest. The primers are then generated by automated DNA synthesis. Because primers may be defined to hybridize to any part of the gene, conditions may be created such that mismatches in the complementary base pairing may be tolerated. Amplification of these mismatch regions may lead to the synthesis of a mutagenized product resulting in the generation of a polypeptide with new properties (site-directed mutagenesis). Also, by coupling complementary DNA (cDNA) synthesis, using reverse transcriptase, with PCR, RNA may be used as the starting material for the synthesis of a recombinant protein of the invention. Furthermore, PCR primers may be designed to incorporate new restriction sites or other features such as termination codons at the end of the segment to be amplified. This placement of restriction sites at the 5' and 3' ends of the amplified nucleic sequence allows for gene sequence including a recombinant protein of the invention or a fragment thereof to be custom designed for ligation with other sequences and/or cloning sites in vectors.

PCR and other methods of amplification of RNA and/or DNA are well known in the art and may be used according to the present invention without undue experimentation. Known methods of DNA and RNA amplification include polymerase chain reaction and related amplification processes (Innes et al., PCR Protocols: A Guide to Method and Amplification) and RNA mediated amplification which uses antisense RNA to the target sequence as a template for double stranded DNA synthesis (US Patent 5,130,238). In an analogous fashion, a recombinant protein of the invention being composed of components 1, (2) and 3 as defined above may be prepared, whereby components 1, (2) and 3 are ligated on a genetic level forming a DNA sequence of the invention, which is used to express a recombinant protein of the invention in a suitable host system.

Also provided by the present invention are vectors encoding the above recombinant protein, and analogs, fragments or derivatives of the invention, which contain the above DNA sequence of the invention these vectors being capable of being expressed in suitable eukaryotic or prokaryotic host cells (claims 9, 10). Particularly preferred are vectors of the invention, which are capable of being expressed in cells of the species *Dictyostelium* (claim 11).

In this embodiment (expression vector) of the present invention the DNA sequence is operably linked to a promoter, preferably linked upstream. The promoter will preferably be an eukaryotic promoter, particularly a constitutive promoter. The transcription of a DNA sequence according to the invention in cells of higher eukaryotes may be derived from viral genomes. Examples would be polyoma viruses, retroviruses, adenoviruses, cytomegaloviruses, SV40 and the like. With mammalian cells a possibility would be the β-actin promoter. In the current invention, the actin15 promoter is particularly preferred for expression in *Dictyostelium.* If appropriate, other regulating elements of transcription and/or translation will be provided. Particularly preferred are cis-acting elements, like enhancer sequences, which usually include 10 to 300 base pairs and act upon the promoter to raise the transcription rate. They may be arranged in the 3' or 5' position of the DNA sequence according to the invention, but also in the coding sequence itself or in an intron sequence which is cut out by splice procedures. Further regulating elements may serve to regulate transcription termination, so that the expression of mRNA is involved.

If necessary, the expression vector with the DNA of the invention are developed as shuttle vectors, that is, they are able to replicate in a host system and can then be transfected into another host system for purposes of expression. For instance, a vector can first be cloned in E.coli and then be inoculated into Dictyostelium, yeast or any mammalian cell for expression.

Typically, such expression and cloning vectors include at least one selection gene exercising a marker function. This is a gene allowing host cells to survive or grow after being transformed by the vector. Typical selection genes code for proteins which permit resistance toward antibiotics or other toxins. This, for instance, includes puromycin, ampicillin or neomycin.

Host cells, particularly eukaryotic host cells, transformed with an expression vector according to the invention are another subject of the present invention (claim 12). Appropriate host cells for cloning or expressing the DNA sequences are prokaryotic cells, yeast or higher eukaryotic cells. In a preferred embodiment, cells for expressing DNA sequences according to the invention are selected from multicellular organisms. This also takes place before the background of the function of component (1) of the recombinant protein of the invention to elements of the cytoskeleton (like actin or microtubules or even components of the cell membrane or membrane of any intracellular organelle, like mitochondria). In principle every eukaryotic cell can be used as host cell, even though cells of mammals, like monkeys, mice, rats, hamster or humans, are preferred. Particularly preferred are cells from the species Dictyostelium (claim 13).

The present invention relates in a further aspect to a method for producing a recombinant protein according to the invention, whereby the method comprises the following steps: (a) preparing a vector according to the invention, (b) transforming eukaryotic host cells with a vector obtainable from step (a), and (c) growing transformed host cells of the invention and obtainable from step (b) under conditions suitable for the expression of said recombinant protein (claim 14). The expression method of the invention allows for overexpression of any target protein or polypeptide of at least 20 amino acid length (component (2)) as segment of the recombinant protein of the invention. Accordingly, huge amounts of target protein as part of a recombinant protein of the invention are produced by the method of the invention. It is preferred within the scope of the present invention to concentrate the overexpressed recombinant protein in the cell. This is achieved by constructing recombinant proteins of the invention, which do not carry any leader sequences for secretion out of the transformed host cell.

Another aspect of the present invention is a method for purifying a recombinant protein according to the invention or any other recombinant protein, which contains an amino acid sequence binding to cytoskeleton (actin or microtubules or proteins being bound to actin in the cell) or membrane (e.g. inner cell membrane or outer or inner membrane of a cell organelle) structures and another amino acid sequence (target sequence to be analysed), whereby the method comprises (a) preparing a vector according to the invention or a vector encoding for any recombinant protein (as disclosed above), (b) transforming eukaryotic host cells with a vector obtainable from step (a), (c) growing transformed host cells according to the invention and/or obtainable from step (b) under conditions suitable for the overexpression of said recombinant protein, (d) purifying overexpressed recombinant protein by binding to endogenous elements or structures of the cytoskeleton or membrane, like actin/microtubules, of the eukaryotic host cell, and (e) releasing bound recombinant protein from these structures or elements, preferably actin or microtubules (claim 15).

In apreferred embodiment of said method for purifying of the invention provides in step (e), the releasing step, a separation from the structures or elements of the cell by adding a substrate, be it a natural or non-natural substrate, of component (1) of the recombinant protein (claim 16). Whereas in general the natural substrate will be used, it may be preferable in certain cases to use a non-natural substrate of component (1), like GTP or (nucleotide) analogues (where ATP is the natural substrate), for releasing purposes. In general, any substrate with the potential to release the bound recombinant protein, particularly by binding to the component (1) of the recombinant protein from the cell structure or element is suitable to be used for step (e). It will be appreciated that a method of the invention using a member of the kinesin or myosin superfamily or a derivative, fragment or analog thereof as component (1) is particularly preferred, if it is characterized in the addition of ATP, which is the natural substrate for these proteins with motility function (claim 17).

In a still further preferred embodiment, the purification method of the invention comprises an additional step (f). Step (f) may typically provide at least one additional in vitro purification step, whereby all common purification procedures available may be provided, for instance all procedures described by A. Mc Pherson (Crystallization of Biological Macromolecules, Cold Spring Harbor Laboratory Press, NY, 1999), which is incorporated herein by reference. In a non-limiting manner, the following methods, particularly biocxhemical and/or physical methods, may be used or combined: salt fractionation, desalting, fractionation with organic solvents or with other precipitants, selection with heat/pH, centrifugation, chromatographic methods, e.g.ion exchange chromatography, molecular sieve chromatography, adsorption chromatography, affinity chromatography or HPLC, ultrafiltration, isoelectric focussing and/or electrophoresis by biochemical, particularly chromatographic, and/or physical methods (claim 18). Affinity chromatography is particularly preferred, whereby metals (e.g. Ni) and/or antibodies are typically bound to a resin as ligands (claim 19). The affinity chromatography may typically be carried out in batch mode or by a column packed with an insoluble support matrix.

A further aspect of the present invention is a recombinant protein, particularly in isolated and/or purified form, obtainable from a method for producing of the recombinant protein of the invention as described herein (claim 20).

A still further aspect of the present invention is a method for crystallizing a recombinant protein according to the invention characterized in that it comprises (a) a purification step according to a method of the invention and (b) a crystallization step (claim 21). Hereby, the purified recombinant protein obtained in step (a) is crystallized by any method known by the skilled person. The crystallizing step will be carried under conditions suitable for crystal growth. The conditions may be optimized by varying certain parameters, such as stock solution, concentration of the recombinant protein, temperature, pH, ionic strength, precipitating agent (e.g. ammonium sulfate or PEG), addition of small amounts of organic solvents etc. However, the conditions used for crystallization of component (1) alone are preferred, which means that the conditions suitable for a member of the myosin or kinesin superfamily or a fragment, analog or derivative thereof may also work to identify crystals of the recombinant protein of the invention. In order to accelerate the crystallization process, it is particularly preferred to apply a recombinant protein of the invention containing as component (1) an amino acid sequence with a flexible region, particularly a flexible at C-terminal end of component (1). Thereby, a high degree of flexibility of the components is achieved resulting in numerous conformations which can be occupied or sampled by the components in the course of the crystallization process.

It is preferred to employ vapor diffusion techniqes either by the hanging or the sitting drop method to obtain crystals. Furthermore, crystallization may be achieved by induction of nucleation. Macro- or microseeding methods are mentioned as described by A. Mc Pherson (Crystallization of Biological Macromolecules, Cold Spring Harbor Laboratory Press, NY, 1999), which is - by its whole contents - incorporated as disclosure of the present application by reference.

Another aspect of the present invention is a protein crystal characterized in that the crystal is built by a network of recombinant proteins according to the invention (claim 22). This network forms the crystal lattice. Within the scope of the present invention are crystals of any space group in which identical proteins can be arranged. A crystal of the invention may contain one, two, three or more recombinant proteins per asymmetric unit. At least one heavy atom may be located at (a) certain position/s in the recombinant protein being arranged symmetrically in the crystal of the invention. Crystals may contain ligands non-covalently bound to the crystallized recombinant protein as well, e.g. ATP, inhibitors, alkali ions or physiological ligands, such as hormones, carbohydrates, protein fragments etc.

Finally, an aspect of the present invention is a method for elucidating the atomic structure of a protein crystal of the invention, whereby, after a crystallization step (a) according to the invention, X-ray diffraction data are collected on a beamline or any kind of device suitable for measuring locations of X-ray reflections (diffractometer, (b)). In final step (c), the atomic structure or rather the electron density map (into which the polypeptide chain and, eventually, other ligands and water molecules are modeled) of a recombinant protein is calculated by Fourier transformation of the data set obtained in step (b) using phasing information obtained by anomalous scattering, the heavy atom method or molecular replacement techniques (claim 23), as e.g. described by Stout & Jensen (X-ray Structure Determination, Wiley, NY, 1989), which is incorporated herein by reference. For the present invention molecular replacement methods are particularly useful. The phasing information may be obtained from component (1) as starting model, which is typically a structurally well determined polypeptide. Therefore, component (1) is a "helper" sequence providing the starting information to solve the structure of the recombinant protein or the structure of component (2), respectively, which is the target protein to be structurally analysed. Further rounds of structure refinement by methods known by the skilled person or described by Stout & Jensen may serve to improve the structure model. Additionally, heavy atoms may be bound to known sites of component (1) of the recombinant protein of the invention. Thereby, additional phasing information may be obtained for structure elucidation of target component (2) (which is under analysis) of the recombinant protein of the invention.

The use of a recombinant protein of the invention for purification and crystallization purposes has unprecedented advantages over the methods known in the art. The recombinant protein via its component (1) binds to insoluble components of the cell, like the cytoskeleton, membrane components or the like. Following cell lysis, the recombinant fusion protein (or rather its component (2), which is wanted to be purified, analysed or subjected to X-ray analysis) can be enriched by ligand depletion and precipitation with the insoluble interaction partners of the cell. This allows for a purification step already carried out in the cell without any additional. Therefore, it is not the lysate as a whole which contains the overexpressed protein but the pre-purified precipitate itself. The specific solubilization of the fusion protein is achieved by addition of the ligand to the insoluble fraction. For crystallization, the conditions (parameters) are preferably chosen such that they coincide with the conditions for structurally well characterized component (1). These conditions or subtle variations of these conditions are expected to work for the recombinant protein as well. Hence, the method of the present invention for crystallizing allows to find crystallization conditions without extensive search for suitable parameters required by the art.

However, it is within the scope of the present invention that a recombinant protein of the invention or any other recombinant protein which is purified according to a method of the present invention may be structurally analysed by any other method known by the skilled person. Particularly, such recombinant proteins may be subjected to NMR analysis (two-dimensional or multidimensional) as described by Roberts (1993, NMR of Macromolecules: A practical approach, Oxford-New York), which is incorporated herein by reference. Furthermore, the system of the present invention may be used for drug design (ligand to component (2) of the recombinant protein used) as described by Craik (1996, NMR in drug design, CRC Press, Boca Raton), which is incorporated by reference. Other methods of structure eclucidation are, for instance, mass spectrotometry as described by Siuzdak (1996, Mass Spectrometry for Biotechnology, Academic Press, San Diego), incorporated herein by reference.

Another object of the present invention is the following subject-matter, namely a method for isolating and identifying proteins which are capable of binding to the target sequence used as component (2) in the recombinant protein (particularly of the invention). Therefore, a yeast-two-hybrid system may be used, by which a sequence encoding the recombinant protein is carried by one hybrid vector and sequence carried by the second hybrid vector, the vectors being used to transform yeast host cells and the positive transformed cells being isolated, followed by extraction of the said second hybrid vector to obtain a sequence encoding a protein which binds to said recombinant protein directly or indirectly via other proteins.

Furthermore, according to the present invention, an approach/method suitable for the identification of binding partners to the recombinant protein of the invention may comprise the following steps: (a) a library of cDNA is typically fused to the C-terminus of component (1), particularly of a myosin motor domain (MMD), (typically resulting in a recombinant protein of the invention), eventually via a linker sequence; (b) this recombinant protein is expressed in *Dictyostelium* or another eukaryotic system; (c) clonal transformants are probed with the bait-protein of choice fused to any marker protein, e.g. β-galactosidase; and (d) after washing identification and determination of interacting recombinant protein by measuring the activity of bait-marker fusion protein, e.g. by addition of β-gal. In a preferred embodiment, the myosin motor domain may be his- or epitope-tagged at the N-terminus. Typically all steps of the method of the invention are carried out in microtiter well plates.

Preferably, the recombinant protein shown to have bound to the bait-protein of choice may be purified by the methods of the invention and can then be subjected to further biochemical or structural characterization, e.g. crystallization as described above, with or without cleavage by a protease, if a recognition in a linker region has been provided, in order to release component (2), the target protein. The method of the invention is suitable for the identification of unknown binding partners and may also be used to demonstrate the interaction between two known polypeptides.

The method of isolating yet unknown binding partners of the invention has numerous advantages over the method known in the art. E.g. MMD-fusion proteins may be easily purified from Dictyostelium and the MMD fusion system may be transferred to a wide range of high eukaryotic cells.
Furthermore, (i) the MMD-cDNA constructs may be directly used for expression in Dictyostelium and other eukaryotic cells; (ii) decreased background (since the system works with purified proteins and not with proteins within a cellular environment that, as in the case of the yeast 2-hybrid-system, leads to a high background of false positive clones); (iii) easy identification and isolation of the positive construct from mother plates; and (iv) the procedure may be highly automated, since all steps in the interaction screening may be performed in microtiter well plates.

### Description of figures

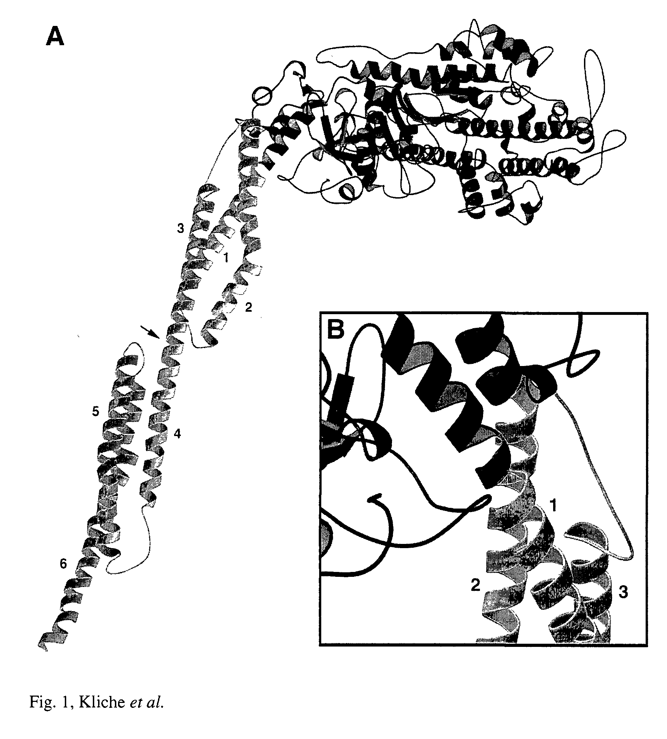

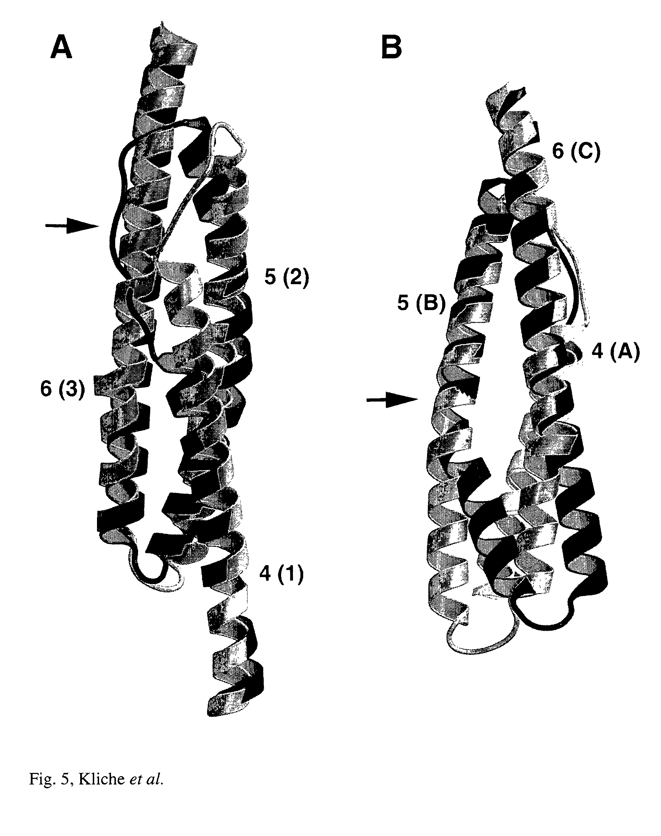

In figure 1 the structure of M761-2R-R238E, an example for a recombinant protein of the invention, is shown. Although two molecules are present in the crystallographic asymmetric unit, only one is shown here. The two molecules are essentially identical throughout the myosin motor domain (residues 2-761) exemplifying component (1) of the recombinant protein of the invention. However, upon leaving the converter domain, the lever arms assume slightly different orientations and deviate at the ends by 19.4 Å. In fig. 1(A) a complete molecule (recombinant protein of the invention) spanning amino acids 2-1010 is shown. No electron density was observed for five residues at the N-terminus, the loop region 205-208, and one residue at the C-terminus. The N-terminal domain (2-200) is shown in green, 50 kDa domain in red (201-613), C-terminal and converter domain in blue (614-761), linker region in orange (762-764) (component (3)) of the recombinant protein of the invention), α-actinin lever arm in yellow (765-1003) (component (2) of the recombinant protein of the invention) and seven histidines from the Hiss purification tag in gray (1004-1010), which are linked as specified for an preferred embodiment of the present invention. The linker region (component (3)) is composed of three residues (Leu-Gly-Arg) introduced during cloning. The observed lever arm is ~140 Å long (measured from Cα of 761 to Cα of 1010). Each α-actinin repeat contributes ~65 Å, and the histidine purification tag another 10 Å. Helices 1-3 make up the first α-actinin repeat, and 4-6 the second. The arrowhead indicates the α-helical region linking the two repeats. The disruptive kink in helix 2 is caused by the presence of two adjacent proline residues (see fig. 5A). In fig. 1(B) a detailed view of the linker region joining the myosin converter domain to helix 1 of α-actinin is depicted. The view is rotated 180° around a vertical axis from that in fig. 1(A).

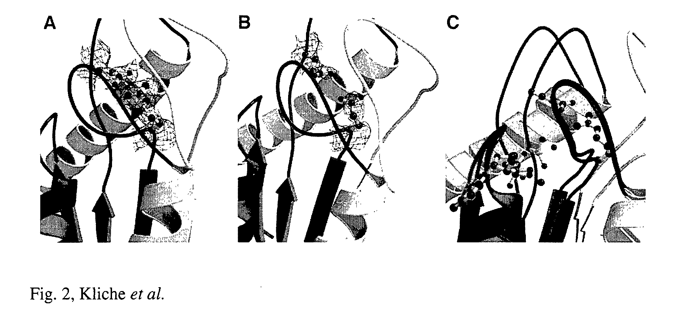

In figure 2 a detailed view of the conserved salt bridge linking switch I and switch II is shown as a result of purifying, crystallizing a recombinant protein of the invention and finally solving the structure of that protein according to methods of the invention. The conserved nucleotide binding/sensing elements found in all myosins, kinesins, and G-proteins are highlighted for the P-loop in blue, switch I in green, and switch II in red. Fig. 2(A) shows the structure of *Dictyostelium* myosin II motor complexed with Mg-ADP-BeF₃. As in Mg-ADP-VO₄ (Smith and Rayment, 1996a) and Mg-ADP-BeF₃(Dominguez *et al.*, 1998) structures, switch I and switch II are closed. The conserved salt bridge between residues R238 and E459 is shown as a ball-and-stick model surrounded by 2.6 Å experimental 2fₒ-f_{c} electron density (blue wire-frame) , contoured at 1 σ. As expected for a salt bridge, the electron density is continuous between the residues, which point toward each other. In fig. 2(B) the same region as observed in the crystal structure of M761-2R-R238E is shown. The electron density was calculated from a model with alanins at positions 238 and 459 in order to eliminate model bias. Electron density for two glutamic acid residues is clearly visible, but the side chain of E238 now points away from E459 and the switch II loop has moved away from switch I. In fig. 2(C) the same region showing a superposition of the M761-2R-R238E structure with a structure of *Dictyostelium,* myosin II motor complexed with Mg-ADP-VO₄ (PDB code 1VOM)(Smith and Rayment, 1996a). The nucleotide and R238-E459 salt bridge are shown as ball-and-stick models. Both the P-loop and switch I regions are in essentially identical conformations in both structures. However, the switch II region (red) shifts to the right, toward the nucleotide, by ~5 Å in the Mg-ADP-VO₄ structure, allowing the formation of the R238-E459 salt bridge.

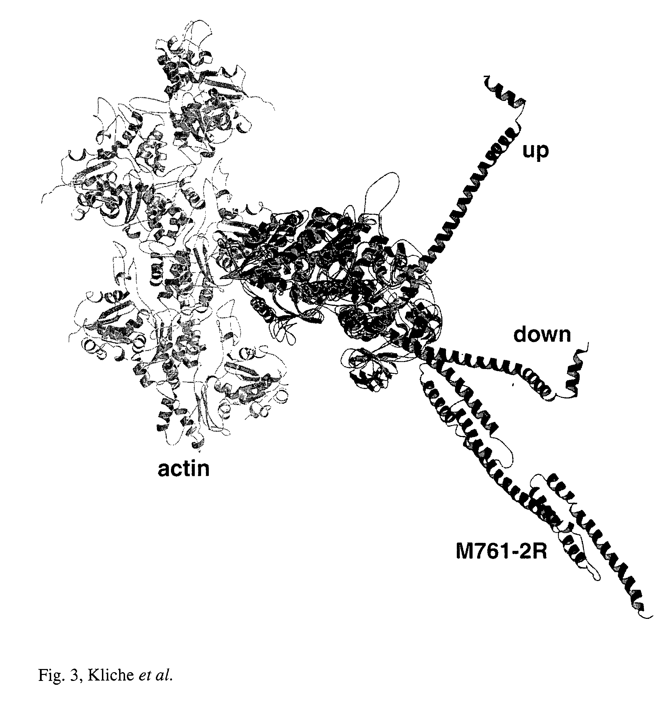

Figure 3 shows the orientation of the myosin lever arm, a segment of component (1) of an example for an recombinant protein of the invention. Shown in yellow are five molecules of actin making up part of a helical actin filament. Modeled on to this are myosin in the "pre-power-stroke" up/closed orientation in red, the "post-power-stroke" down/open orientation in blue, and the M761-2R-R238E structure in green. The up, down, and actomyosin complex structures were modeled. The M761-2R-R238E structure was then aligned to the core domain of the down/open structure via residues 160-200, which includes the highly conserved P-loop region. It is noted that in the M761-2R-R238E structure, the helix leaving the converter domain initially superposes with the down/open structure, but then deviates due to the different helical bend of the α-actinin.

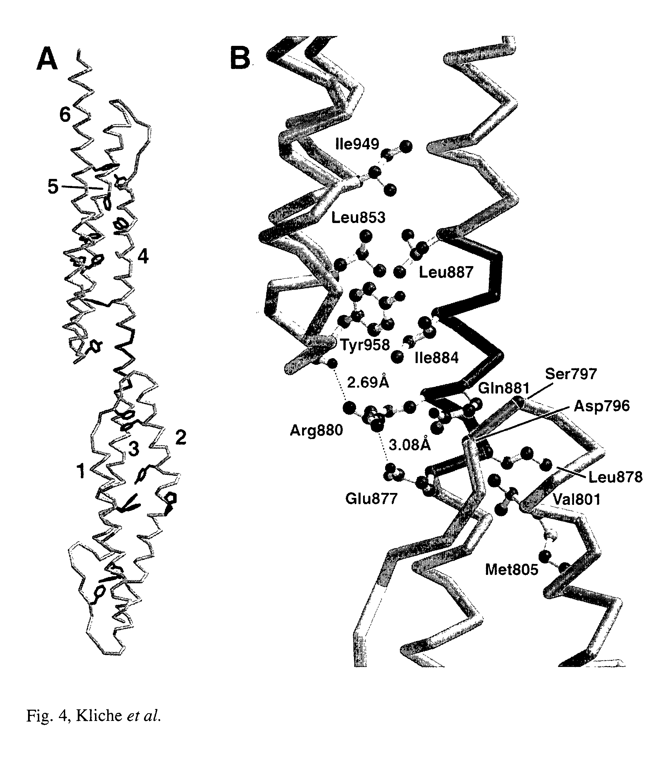

In figure 4 the structure of α-actinin repeats 1 and 2 are shown. α-Actinin is an example for component (2) of the recombinant protein of the invention, which means α-actinin is the target protein in this example. Its strucure was solved using purification and crystallization methods of the present invention. The structure of the fig. 4(A) depicts in yellow an α-carbon chain trace of the 6 helices making up repeats 1 (helices labeled 1-3) and 2 (helices labeled 4-6). The 17 hydrophobic aromatic amino acid residues stabilizing the triple-helical packing are shown in green (7 tyrosines, 6 phenylalanines and 4 tryptophans). Shown in red are two adjacent proline residues, which cause a kink, but not a break in α-helix 2 of repeat 1. The uninterrupted α-helix linking repeats 1 and 2 is shown in orange. Fig. 4(B) exemplifies a detailed view of the linker region, highlighting the stabilizing hydrophobic and hydrogen bonding interactions. Colours and orientation are identical to those in fig. 4(A). Side chains are shown as ball-and-stick models, with the exception of Asp796 and Ser797, in which only the α-carbon atoms involved in hydrophobic contacts are shown for clarity. The salt bridge between Arg880 and Glu877, and the hydrogen bond between Arg880 and the carbonyl oxygen of Leu956 (also shown as a ball-and-stick model), are shown as dashed lines.

Figure 5 is a comparison of *Dictyostelium* α-actinin with human α-actinin and human α-spectrin. Fig. 5(A) shows the overlapping repeat 2 region of *Dictyostelium* (yellow) and human (blue) α-actinin are shown as ribbon diagrams. Helices are numbered as described above for *Dictyostelium* α-actinin and, in parentheses, as described previously for human α-actinin (Djinovic-Carugo *et al.,* 1999). The largest differences occur in the loop region connecting helices 4 and 5, indicated by an arrow, where the human α-actinin structure would seriously overlap with *Dictyostelium* helix 6. In fig. 5(B) the alignment of *Dictyostelium* repeat 2 (yellow) with repeat 16 human α-spectrin (green) is shown as ribbon diagrams. Helices are numbered as described above for the *Dictyostelium* protein and, in parentheses, as described previously for the human protein (Grum *et al.,* 1999). *Dictyostelium* helix 4 and α-spectrin helix A, which are in the background, are colored white for clarity. In general, the two structures align more closely than the human/*Dictyostelium* alignment described above. The largest difference occurs in the loop region connecting helices 5 and 6, indicated by an arrow, where the human α-spectrin structure is moved in respect to the *Dictyostelium* α-actinin structure as a result of a proline-induced kink in helix B.

Fig. 6 shows the amino acid sequence (one-letter-code) for component (1) of recombinant protein M761-2R R238E exemplifying a recombinant protein of the invention.

In fig. 7 the whole sequence of recombinant protein M761-2R R238E is depited comprising as component (1) the amino acid sequence of the myosin II motor domain of Dictyostelium, a three amino acid linker region ( ) as component (3) and the a-actinin amino acid sequence being the target sequence in this example (one-letter-code). Fig. 8 is the DNA sequence coding for recombinant protein M761-2R R238E such that the sequence of fig. 8 corresponds to the sequence of fig. 7 on the genetic level.

### Examples of the present invention

### Example 1

### (a) Expression

The expression-vector pDXA-3H, that was used for the production of M761-2R R238E, carries the origin of replication of the *Dictyostelium* high copy number plasmid Ddp2 (Leiting *et al.* 1990, *Molecular And Cellular Biology* **10,** 3727-3736; Chang *et al.* 1990, *Nucleic Acids Research* **17,** 3655-3661), an expression cassette consisting of the strong, constitutive *actin*15 promoter, a translational start codon upstream from a multiple cloning site (MCS), and sequences for the addition of a histidine octamer at the carboxy terminus of any protein. Plasmids derived from pDXA-3H were transformed into orf⁺-cells. These cells carry several integrated copies of the *rep* gene which is essential in *trans* for the replication of plasmids that carry the Ddp2 origin (Leiting *et al.* 1990, *Molecular And Cellular Biology* **10,** 3727-3736; Slade *et al.* 1990, *Plasmid* **24,** 195-207). The myosin-α-actinin fusion was created by linking codon 761 of the *Dictyostelium mhcA* gene to codon 264 of the *Dictyostelium α-actinin* gene.

The resulting construct pDH12-2R extended to codon 505 of the α-actinin gene. Plasmid pDH20 was generated by insertion of the first 765 codons of Dictyostelium myosin II into the MCS of pDXA-3H (Furch et al. 1998, *Biochemistry,* **37,** 6317-6326). Site directed mutagenesis was used to generate plasmid pDH20(R238E) encoding a motor domain fragment with the single point mutation R238E. Replacement of the 2 kb SafI-BstXI fragment of pDH12-2R with the corresponding fragment from pDH20(R23BE) was used to generate the expression vector for the production of M761-2R R238E, the fusion protein and an example for a recombinant protein of the invention, containing thus both a point mutation in the active site and a C-terminal extension consisting of two α-actinin repeats.

### (b) Purification

The overexpressed protein was purified by Ni²⁺-chelate affinity chromatography as described by Manstein and Hunt (J. Muscle Res. Cell Motil., 1995, 16, 325) und Manstein et al. (Gene 1995, 162, 129). Both afore-cited documents are incorporated into the disclosure of the present invention by their complete contents.

Cells expressing the histidine octamer tagged fusion protein were grown in 5 l flasks containing 2.5 l DD-Broth 20. DD-Broth 20 contains (per litre): 20 g protease peptone (Oxoid), 7 g yeast extract (Oxoid), 8 g glucose, 0.33 g Na₂HPO₄.7H₂O, and 0.35g KH₂PO₄. The flasks were incubated on a gyratory shaker at 200 rpm and 21°C. Cells were harvested at a density of 6 x 10⁶ ml⁻¹ by centrifugation for 7 min at 2,700 rpm in a Beckman J-6 centrifuge and washed once in phosphate buffered saline. The wet weight of the resulting cell pellet was determined. Typically, 35 g were obtained from a 15 l shaking culture. The cells were resuspended in 140 ml of Lysis Buffer (50 mM Tris.HCl, pH 8.0, 2 mM EDTA, 0.2 mM EGTA, 1 mM dithiothreitol (DTT), 5 mM benzamidine, 40 mg/ml TLCK, 20 mg/ml N-tosyl-L-phenylalanine chloromethyl ketone (TPCK), 200 mM phenylmethylsulfonyl fluoride (PMSF), 0.04 % NaN₃).

Cell lysis was induced by the addition of 70 ml of Lysis Buffer containing 1 % Triton-X100, 15 mg/ml RNaseA (Sigma) and 100 units of alkaline phosphatase. The lysate was incubated on ice for one hour. Upon centrifugation (230,000*g*, 1 hour), the recombinant protein remained in the pellet. The pellet was washed in 100 ml of HKM buffer (50 mM HEPES, pH 7.3, 30 mM KAc, 10 mM MgSO₄, 7 mM b-mercaptoethenol, 5 mM benzamidine, 40 mg/ml PMSF) and centrifuged for 45 min at 230,000*g*. The recombinant protein was released into the supernatant by extraction of the resulting pellet with 60 ml HKM buffer containing 10 mM ATP. After centrifugation (500,000g, 45 min.), the supernatant was loaded using a peristaltic pump onto a Ni²⁺-nitrilotriacetic acid (Ni²⁺-NTA) affinity column (1.5 x 10 cm) (Qiagen). The flow-rate was adjusted to approximately 3 ml min⁻¹. After loading was completed the column was connected to a Waters 650M chromatography system. The column was washed briefly in Low Salt buffer (50 mM HEPES, pH 7.3, 30 mM KAc, 3 mM benzamidine), High Salt buffer (as Low Salt Buffer, but with 300 mM KAc), and Low Salt Buffer containing 50 mM imidazole. the recombinant myosin was eluted using a linear gradient of Low Salt Buffer and Imidazole Buffer (0.5 M imidazole, pH 7.3, 3 mM benzamidine), starting with 10 % Imidazole Buffer and reaching 100 % after 15 minutes. The flow rate was 3 ml min⁻¹ and 3 ml fractions were collected. Absorbance at 280 nm was monitored. SDS gels were run to check the purity of the eluted protein.

The pooled fractions were dialysed immediately against Storage Buffer (20 mM HEPES, 0.5 mM EDTA, 1 mM DTT, pH 7.0) containing 3% sucrose and the purified protein could be stored at -80°C for several months without apparent loss of enzymatic activity. Actin-activated ATPase activity was measured by the release of inorganic phosphate.

### (c) Crystallization

Crystals of the overexpressed and purified recombinant protein M761-2R R238E were grown by the hanging drop method at 7°C. The drops contained equal volumes (2.2 µl) of the protein solution and the mother liquor. The mother liquor contained 12% PEGM 5K. 170 mM NaCl, 50 mM HEPES-NaOH pH 7.2, 5 mM MgCl₂, 5 mM DTT, 0.5 mM EGTA and 2% 2-methyl-1,3-propanediol. The protein solution (5 mg/ml) contained additionally 200 µM ADP and 200 µM vanadate, and was incubated on ice for 1 h before setting up the drops. Crystals normally appeared after 7-8 days and reached maximum dimensions of 0.1 x 0.3 x 0.9 mm. Crystals were transferred to a solution of mother liquor plus 30% glycerol and frozen in liquid nitrogen for storage and data collection.

### (d) Crystallography and structure refinement

Diffraction data for the crystals of the recombinant protein M761-2R R238E were collected at ESRF beamline ID-13 on a MarCCD detector and integrated and scaled using the program XDS (Kabsch, 1993, J. Appl. Cryst., 26, 795), producing a data set 97.7% complete to 2.8 Å with 4-fold redundancy and an R_{sym} of 11.0%. The M761-2R-R238E crystals belonged to space group P2₁2₁2 with two molecules in the asymmetric unit. Molecular replacement was performed with the program AMoRe (Navaza, 1994, Acta Cryst. A 50, 157) using the crystal structure of Dictyostelium myosin resides 2-759 complexed with Mg-ADP-BeFₓ (PDB code 1mmd) (Fisher et al., 1995, Biochemistry, 34, 8960) as a starting model (the nucleotide and the side chains beyond Cβ of residues 238 and 459 were excluded).

Initial maps showed clear helical density for the first repeat of the α-actinin lever arm, which was built as a poly-alanine model using the program O (7.0 for WindowsNT). Following several rounds of simulated annealing refinement using torsional dynamics and a maximum likelihood target with the program CNS v0.9a (Brünger et al., 1998, Acta Cryst. D, 54, 905), the second α-actinin repeat was visible and built. Subsequent rounds of model building and refinement (including bulk solvent correction) produced the final structure of two M761-2R-R238E molecules containing 1005 residues each, two molecules of Mg-ADP and 14 water molecules (R-factor, 24.1%; R_{free}, 29,9%). Ramachandran analysis shows all non-glycine residues to be in allowed regions. Figures were made using the programs Bobscript (Esnouf, 1997, J. Mol. Graph. Model., 15, 132) and Raster3D (Merritt and Bacon, 1997, Methods Enzymol., 277, 505).

### Example 2

Myosin-Fusion-System for isolating interacting proteins/protein binding partners

### (a) Preparation

In order to demonstrate the function of the myosinfusion-system a library of cDNA was fused to the C-terminus of a myosin motor domain (MMD) and expressed in *Dictyostelium* or another eukaryotic system. Clonal transformants were probed with the bait-protein of choice fused to β-galactosidase. The myosin motor domain was His- or epitope-tagged at the N-terminus.

Experimentally, cells were transformed with the MMD-cDNA library and clones were grown and kept in 96 wells plates. The bait-β-gal fusion protein was transformed in *Dictyostelium* Orf+ cells and grown in an appropriate quantity (1 clonal cell line). Upon reaching confluence, the MMD-cDNA clones in the 96 wells plates were washed once in the plates with PBS and then lysed by adding lysis buffer containing Triton X-100 (or, alternatively, NP-40), at the same time the ATP pool was depleted by the addition of alkaline phosphatase. The actin-based cytoskeleton with all myosin and also the M765-fusion-proteins were pelleted by centrifugation and washed with lysis buffer. The myosin was released from the pellets by the addition of Mg²⁺-ATP. The ATP-unsoluble fraction was pelleted and the supernatant transferred to 96 wells plates coated with Ni-NTA. The His-tagged products of the MMD-cDNA were shown to bind to these plates. After extensive washing the coated plates were incubated with the bait-β-gal construct. Again, after extensive washing the plates were incubated with a substrate for β-gal, in this case CPRG (red color OD₅₇₄) or ONPG (yellow OD₄₁₅), and the β-gal activity was determined with a microtiter plate reader. High β-gal activity indicated a strong interaction between the bait and the product of the target cDNA.

The selected clones were then recovered from the original 96 wells plates. The MMD-cDNA-clone was expressed in and purified from *Dictyostelium* by standard myosin motor domain purification. For further biochemical and structural characterization, the isolated gene product was either cleaved with an appropriate protease to release it from the MMD or was used directly in the fusion form for kinetics or crystallization experiments.

### (b) Interaction Test

The method of the invention was tested by expressing MMD-Rac1A and DRG-2D-β-gal (the DRG-2D construct acts as an exchange factor for the small G-protein Rac1A).

The MMD-Rac1A cells were cloned, grown in 96 wells plates, washed, lysed and ATP extracted as described above. The Ni-NTA coated plates were then incubated with the ATP-released protein fraction. The cells expressing the DRG-2D-β-gal were grown in shaking suspension and washed and lysed under the same conditions. The DRG-2D-β-gal supernatant was incubated at different dilutions. As controls wells were incubated without bait (DRG-2D-β-gal) or without MMD-Rac1A or with MMD alone. All controls were negative after staining for β-gal, whereas the incubations with immobilized MMD-Rac1A and the bait gave a signal, which was dependent on the concentration of added bait.

In conclusion, the interaction between DRG-2D and Rac1A was shown by the method of the invention, whereas it could not be shown when using the yeast two-hybrid system. Therefore, the method of the invention has definitely advantages over the yeast-two-hybrid system or other techniques developed to identify protein-protein interactions and known in the art.

## Claims

1. Recombinant protein **characterized in that** it contains as component (1) an amino acid sequence of a member of the myosin or kinesin protein superfamilies or an amino acid sequence of an analog, fragment or derivative of a member of the myosin or kinesin protein superfamilies, as component (2) any amino acid sequence of at least 20 amino acids in length, and as component (3) a linker region of at least 2 amino acids between components (1) and (2).

2. Recombinant protein according to claim 1 **characterized in that** it contains as component (1) an amino acid sequence of a member of the myosin I, II, III, IV, V, VIII, VI, X, or XI or a member of kinesin I or II families or an amino acid sequence of an analog, fragment or derivative of a member of the aforementioned myosin and kinesin families.

3. Recombinant protein according to claim 1 or 2 **characterized in that** it contains as component (1) an amino acid sequence for the motor domain of a kinesin or myosin family member or an analog, fragment or derivative thereof.

4. Recombinant protein according to any of the preceding claims **characterized in that** it contains as component (3) a stretch of 3 amino acids with a Gly in the middle.

5. Recombinant protein according to any of the preceding claims **characterized in that** it contains as component (2) the sequence of an esterase, hydrolase, phosphatase, kinase, protease, channel, structural protein (e.g. coronin, spectrin), receptor, particularly a neuronal or immunologically relevant receptor (e.g. superfamily of TNF receptors), transcription factor, DNA/RNA-binding protein, lipoprotein or glycoprotein or an anlog, derivative or fragment thereof.

6. Recombinant protein according to any of the preceding claims 1 to 5 **characterized in that** it contains as component (1) an amino acid sequence as shown in figure 6 and as component (3) the linker amino acid sequence Leu-Gly-Arg.

7. Recombinant protein according to any of preceding claims 1 to 6 **characterized in that** it contains a tag sequence, particularly at the N- or C-terminus of the recombinant protein.

8. DNA sequence **characterized in that** it contains a sequence which codes for an amino acid sequence according to any of the preceding claims 1 to 7.

9. Vector containing a DNA sequence according to claim 8.

10. Vector according to claim 9 capable of being expressed in a eukaryotic host cell.

11. Vector according to claim 9 or 10 capable of being expressed in cells of the species Dictyostelium.

12. Transformed eukaryotic host cell containing a vector according to any of preceding claims 9 to 11.

13. Transformed eukaryotic host cell according to claim 12 **characterized in that** it is a cell from the species Dictyostelium.

14. Method for producing a recombinant protein according to any of the preceding claims 1 to 7 **characterized in that** it comprises (a) preparing a vector according to any of preceding claims 9 to 11, (b) transforming eukaryotic host cells with a vector obtainable from step (a), (c) growing transformed host cells according to claim 12 or 13 and obtainable from step (b) under conditions suitable for the expression of said recombinant protein.

15. Method for purifying a recombinant protein according to any of preceding claims 1 to 7 **characterized in that** it comprises(a) preparing a vector according to any of preceding claims 9 to 11, (b) transforming eukaryotic host cells with a vector obtainable from step (a), (c) growing transformed host cells according to claim 12 or 13 and obtainable from step (b) under conditions suitable for the overexpression of said recombinant protein, (d) purifying overexpressed recombinant protein by binding to endogenous actin/microtubules (mt) of the eukaryotic host cell, and (e) specifically releasing bound recombinant protein from actin/mt.

16. Method according to claim 15 **characterized in that** step (e) comprises releasing the recombinant protein by adding a natural substrate of component (1) of the recombinant protein.

17. Method according to claim 16 **characterized in that** the natural substrate is ATP.

18. Method according to any of preceding claims 15 to 17 **characterized in that** it comprises as further step (f) at least one additional purification step by biochemical, particularly chromatographic, and/or physical methods.

19. Method according to claim 18 **characterized in that** step (f) comprises purifying by affinity chromatography, particularly using metals or antibodies as ligands.

20. Recombinant protein obtainable from a method according to any of preceding claims 15 to 19.

21. Method for crystallizing a recombinant protein according to any of preceding claims 1 to 7 **characterized in that** it comprises (a) a purification step according to a method according to any of preceding claims 15 to 19 and (b) crystallizing the purified recombinant protein obtained in step (a).

22. Protein crystal **characterized in that** the crystal is built by a network of recombinant proteins according to any of preceding claims 1 to 7 that form the crystal lattice.

23. Method for elucidating the atomic structure of a protein crystal according to claim 22 **characterized in that** it comprises (a) a crystallization step according to claim 21, (b) collecting X-ray diffraction data for a crystal obtained in step (a), and (c) calculating the atomic structure of a recombinant protein by transformation of the data obtained in step (b) .
